# EUROPEAN PATENT SPECIFICATION

(11) **EP 3 531 126 B1**
(45) Date of publication and mention of the grant of the patent: **07.04.2021**
(21) Application number: 19159499.3
(22) Date of filing: 26.02.2019
(51) Int. Cl.: G01N 33/12, G01N 21/88, A22C 25/04

(54) **METHOD AND APPARATUS FOR THE INSPECTION OF PACKAGED FISH PRODUCTS**
VERFAHREN UND VORRICHTUNG ZUR INSPEKTION VERPACKTER FISCHPRODUKTE
PROCÉDÉ ET APPAREIL D'INSPECTION DE PRODUITS DE POISSON EMBALLÉS

(30) Priority: 27.02.2018 IT 201800003091
(43) Date of publication of application: 28.08.2019
(73) Proprietor: NORDFISH S.R.L., 20138 Milano (IT)
(72) Inventor: ZOTTI, Francesco, 33033 Codroipo (IT)
(74) Representative: Petraz, Gilberto Luigi

(56) References cited:
- WO-A1-2007/128316
- JP-A- 2004 045 072
- US-A1- 2015 268 211
- US-A1- 2017 205 385

## Description

### FIELD OF THE INVENTION

The invention concerns a method and an apparatus for the inspection of food products, in particular of packaged raw fish products, generally used to check the possible presence of parasites, bones, or other foreign bodies in products intended for consumption without cooking.

The present invention also concerns a method of inspection of food products, in particular packaged raw fish products, to identify the possible presence of parasites.

### BACKGROUND OF THE INVENTION

Devices are known, which are used to inspect food products to check the possible presence of parasites and infestant organisms in them.

In the specific sector of fish products, special attention is required in inspections because of the increasing popularity of raw fish, and the food chain that connects this food with human beings can cause the transmission to the organisms of the latter of parasites and infestants which can be found in the flesh of raw fish products intended for food, both in the form of larvae as well as in the developed form.

Typically, inspections are performed by disposing the food samples, such as for example portions in the form of thin fillets, resting them on a support, for example a conveyor belt, and irradiating them with beams of light that allow an observer to detect with the naked eye the possible presence in the samples of shaded areas, more or less evident, in which the parasites can potentially nest.

In the particular case of fish products, the inspections are intended to exclude the presence of a parasite known as Anisakis.

This parasite occurs in the adult state in the form of a thin worm that nests in fish flesh and which, if ingested, takes root in the human body, typically the stomach mucosa, generating a pathology called anisakidosis or anisakiasis in the human. The worm is killed by freezing or cooking so it is not a danger for food frozen at -18°C for more than 96 hours or cooked at 60°C for one minute. In the case of fish products intended for consumption without cooking, therefore, long abatement times are required, which entail problems of logistics and equipment and considerable energy consumption, which considerably affect the cost of the final product. Furthermore, freezing causes the breakdown of nutrients, making the product more perishable once thawed, as well as less juicy and tasty.

In the sector of consumption of food products, devices are known to inspect fish products or meat during processing.

Such machines, used until now in the control of fish products, provide to use supports on which the samples to be inspected are positioned, and devices to illuminate the products with backlight and obtain an image of them.

Photographic shots or videos, taken from one or more angles and from one or more sides, can be projected onto a monitor.

Document WO-A-2007/128316 describes a method and an apparatus which provide to pass a light beam through an object, in particular a food product, making it advance on a conveyor belt and illuminating it from one side with a light source which has a narrow band cross section, which is received by a camera which receives and photographs the illuminated narrow band portion of the food product, so as to obtain a scan of the food product as it advances. This solution also provides to detect whether or not a food product is present on the conveyor belt using a pair of detection lights which act in inclined and incident directions with respect to each other, so as to switch off the light source when no food product is present on the conveyor belt.

US-A-5,847,382 describes a device to identify bone fragments in a boned chicken. The device comprises a conveyor belt on which the boned chickens are made to advance, a fluorescent light source located under the conveyor belt, and a second conveyor belt disposed above the first belt, which presses the boned chickens to reduce their thickness, and inside which a video camera is disposed used to frame the flattened portions of chicken and identify possible bone fragments.

JP-A-H01 111922 describes an apparatus to identify the type and size of a fish by recognizing the characteristics of the surface and of the shape of the pectoral fin. The apparatus comprises two chambers, of which one chamber is provided with a light source facing toward a conveyor belt on which the fish advance, and a video camera which detects the light image reflected by the illuminated fish, and a second chamber provided with a light source located below the conveyor belt and a video camera located above to identify the shape of the fish. This apparatus, however, does not allow to identify possible parasites inside a fish fillet.

One disadvantage of known solutions is that the samples are inspected only on the side which, in the inspection apparatuses, is facing toward the light sources which irradiate it; this circumstance, when the samples have a not inconsiderable thickness, can prevent the detection of parasites that nest in proximity to the opposite side to the irradiated one and which rests on the support plane.

Therefore, in order to observe both sides, it is necessary to turn the sample over. This operation, however, is possible only if the product has not yet been packaged, or if the package is of the vacuum-packed type, or if the product has undergone a process that has made it more rigid, such as smoking, salting, dehydration or freezing, but it is not possible in the case of products packaged in trays.

The use of take-away and disposable packages, which use trays with a transparent lid that makes it possible for the final consumer to see the raw fish product in fillets or slices, is widespread to the detriment of vacuum packs which are mainly used for smoked or frozen products. This type of article cannot be turned over because the lid would become dirty and ruin the package.

Another disadvantage of known solutions is that, if the samples to be inspected have very limited thicknesses, the irradiation of the light sources and the heat of the lamps can damage the product.

If the light sources used are incandescent, they heat the fresh fish product, while if they are neon, they do not have sufficient intensity to pass through the thickness of the fish fillets and they cannot be switched off and on with speed.

Furthermore, a large number of samples to be analyzed as in an industrial production is not suited to solutions that provide to use glass sheets as transparent support for the sample. To date, the movement of the products on the sheets is manual, with the problem that the operator has a beam of light pointed in his eyes during this operation. In this condition where his field of vision is disturbed, the ability to detect the presence of shadows on the fish is highly compromised.

The solutions that provide to dispose the packages on conveyor belts made of plastic material are ill suited to the operating conditions of a food industry where the frequent sanitizations with aggressive products make the belts opaque, making them inefficient. Furthermore, belts made of plastic material include junction points, which have poor transparency or in any case a different transparency compared with the rest of the belt.

Another disadvantage of known solutions is that the acquisition of the images takes place in such conditions that external reflections can reduce the visibility of the shadows present on contaminated samples, so they do not allow to effectively recognize parasites such as those of the genus Anisakis, which has thin shapes and sizes.

Moreover, in known solutions it is not possible to guarantee the full traceability of the product and the possibility of proving that the sample has been inspected, and therefore they are not suitable to be used to identify parasites in food products intended for consumption of uncooked food.

One purpose of the invention is to improve the state of the art.

Another purpose of the invention is to perfect a method and an apparatus for the inspection of food products, in particular of raw fish products packaged in packages which cannot be turned over, which allows to detect, both quickly and safely, the presence of parasites and other infestant organisms, bones, or other foreign bodies harmful to humans.

Another purpose of the invention is to perfect a method and an apparatus for the inspection of food products that allows to perform inspections on a large number of packages without compromising the quality of the inspection and improving the working conditions and the efficiency of the operator.

Another purpose of the present invention is to provide an apparatus and to perfect a method to inspect food products that also allows to recognize and identify possible parasites located on a side opposite the irradiation side.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

An apparatus for the inspection of food products is provided, suitable in particular to inspect samples of raw fish products packaged in packages which cannot be turned over, for example transparent plastic trays.

The apparatus according to the invention comprises a box-like frame which delimits an inspection chamber, and feed means and removal means configured respectively to feed the samples containing the food products to be inspected into said chamber and to remove the inspected samples from said chamber.

According to some embodiments, the removal means can comprise a conveyor belt and a piston device suitable to remove a package from the conveyor belt and position it on the support plane.

According to possible solutions, other pistons can be provided downstream of the inspection chamber, configured to thrust the previously inspected package onto an exit conveyor belt.

The inspection apparatus also comprises a transparent support plane, disposed in the inspection chamber and aligned with the feed and removal means, which is configured to support at least one package of food products to be inspected.

Preferably, the support plane is made of transparent glass, so as to allow substantially the passage of the entire light beam emitted by the light sources and allow cleaning and hygiene.

The apparatus also comprises at least two light sources disposed on opposite sides of the plane and each configured to emit a light beam toward a package disposed thereon, and at least two visual inspection devices disposed on opposite sides of said plane, each configured to cooperate with one of the light sources to acquire a backlit image of the food product inside the package.

The apparatus includes a processing unit, connected to the visual inspection devices and configured to alternatively activate or deactivate the visual inspection devices and the light sources to allow the acquisition of the images, and to process the images acquired, and a display device configured to display the images acquired, making them available to an operator.

The apparatus also comprises opaque reference elements provided on said plane, able to be acquired by said visual inspection devices together with said backlit images of said food product, wherein said processing unit is configured to generate an overlapping image of said first image and said second image according to said acquired reference elements.

Said opaque reference elements are provided on the support plane, so as to interfere with the light beam emitted by the light sources and be acquired by the visual inspection devices.

In this way it is possible to generate an image given by overlapping the two images acquired above and below the plane, resizing or rotating them in a suitable manner in order to make the respective reference elements match.

Advantageously, the light sources comprise LED devices, so as to limit the generation of heat and prevent possible damage to the food products. Furthermore, the LED devices can be switched on/off in a substantially immediate way, allowing to quickly acquire the images from one and the other visual inspection device, so as to have high efficiency.

According to some embodiments, the box-like frame and the plane substantially delimit a first dark chamber located above the plane and a second dark chamber located below the plane.

According to some embodiments, the visual inspection devices and the light sources are located in opposite and symmetrical positions with respect to the plane. In this way it is possible to obtain a compact apparatus, reducing the bulk and at the same time allowing to obtain an image for each side of the food product, allowing to also identify parasites of small sizes and with thin shapes, or possible bones or other foreign bodies positioned at any height of the thickness of the food.

According to possible variants, the visual inspection devices and the light sources are reciprocally offset in a direction of feed of the packages in the chamber.

The apparatus according to the invention therefore allows to perform a rigorous inspection of raw fish products intended for consumption without cooking, without having to provide for the abatement step, avoiding the disadvantages indicated above and at the same time guaranteeing the safety of the consumer.

A second aspect of the invention concerns a method for the inspection of samples, in particular packaged raw fish products. The method comprises:
- feeding a plurality of samples to be inspected by means of feed means into an inspection chamber delimited by a box-like frame;
- inserting at least one packaged sample in the inspection chamber resting on a transparent plane;
- illuminating the sample with a first light source located underneath the plane and acquiring a first backlit image of the sample contained in the package with a first visual inspection device disposed above the plane;
- illuminating the sample with a second light source located above the plane and acquiring a second backlit image of the food product with a second visual inspection device disposed below the plane;
- processing the images acquired in order to recognize a parasite or foreign body in the food product and make them visible to an operator on a display device.

The method according to the invention provides to also acquire, together with the backlit image of the sample, an image of opaque reference elements provided on the plane and to generate an image given by overlapping the first and second image on the basis of the reference elements.

According to embodiments, the method can provide to read a code provided on a package and carrying information correlated to the packaged sample and, at the end of processing, can provide to digitally memorize the images, match them to the code it has read, and possibly memorize them in a memory unit.

If the inspections detect the presence of parasites, the packages in question are removed from the marketing line and sent to sanitation processes.

The invention allows to obtain the following advantages:
- to perform more accurate inspections on samples of packaged fish products in packages which cannot be turned over to detect the presence of parasites and infestant organisms, or foreign bodies;
- to perform the inspection in the best light conditions, in particular using a glass plate as a support, which has maximum transparency, and a dark chamber to reduce light interference.
- to perform inspections on a large number of samples continuously;
- to perform accurate inspections on both sides of each sample;
- to maintain the state of the samples unaltered;
- to guarantee the operator a better working condition;
- to facilitate the search for the parasite by digitizing the images;
- to provide proof the inspection is completed for each individual package as a guarantee to the consumer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a schematic view of a simplified embodiment of an apparatus for the inspection of food products according to embodiments described here;
- fig. 2 is a schematic view of a part of the apparatus of fig. 1;
- fig. 3 is a lateral schematic view of an inspection apparatus according to embodiments described here;
- fig. 4 is a schematic view from above of the inspection apparatus of fig. 5;
- fig. 5 is a schematic view of an apparatus for the inspection of food products according to variant embodiments;
- fig. 6 is a schematic view of an apparatus for the inspection of food products according to another variant embodiment.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

In the following description, for ease of description we have referred to fish products to be inspected. A person of skill, however, understands that other types of food products can also be inspected according to the method and apparatus according to the invention.

With reference to the drawings, 1 indicates as a whole an apparatus for the inspection of food products, hereafter briefly apparatus 1, in this specific case raw fish prepared in portions, which define the samples 2 to be inspected.

The samples 2 can comprise, for example, both a slice of fish, as well as portions thereof in the form of thin fillets.

According to some embodiments it can be provided that the samples 2 are packaged in containers or trays 25 made of at least partly transparent material to allow the passage of the inspection light through them.

The apparatus 1 comprises a box-like frame 13 which defines an internal chamber 14 in which an inspection of the samples 2 is performed to identify possible parasites in them, for example of the genus Anisakis, or possible bones or other foreign bodies.

A transparent support plane 3 is provided inside the chamber 14, suitable to support at least one sample 2 or a package 25.

The apparatus 1 comprises a light source 6a, disposed below the support plane 3.

The apparatus 1 also comprises a light source 6b disposed above the support plane.

Each of the light sources 6a, 6b typically consists of one or more lamps 7 which emit an inspection light, as shown schematically by arrows F1, F2, facing in the direction of the transparent support plane 3, so as to pass through it and irradiate the sample 2 in the package 25, from the bottom and from the top respectively.

As can be seen, a lamp 7 is located below the transparent plane 3 and irradiates both the latter, as well as the samples 2 in the packages 25, from the bottom upward, making them inspectable through transparency, while another is located above the plane 3 and irradiates the samples 2 from the top downward.

According to some embodiments, the support plane 3 is made of a transparent glass sheet, so that it allows the passage of the entire light beam emitted by the light sources 6a, 6b.

According to some embodiments, the plane 3 can be supported by a base box-like frame 4 which defines an internal compartment 5 in which the lower light source 6a is housed.

A cooling plant 8 can also be provided in the compartment 5 which comprises at least one fan 9 which is rotatably motorized with a motor unit 10, preferably located outside the compartment 5.

According to some embodiments, the fan 9 is of the extractor type and, when it is operating, generates a flow of cooling air, indicated by arrows F2, which first hits the lamp 7 and is then thrust toward an exit pipe 11.

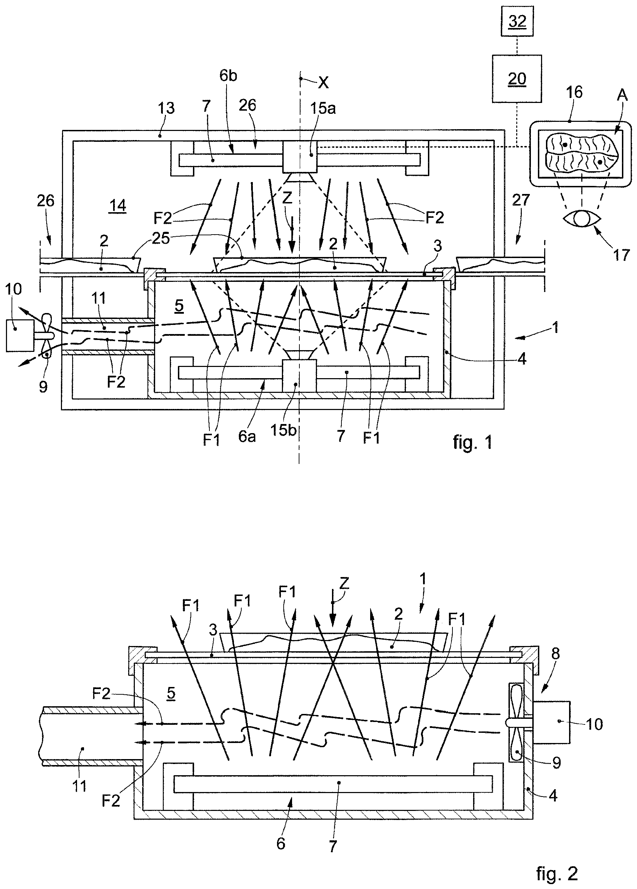

According to variant embodiments, for example described with reference to fig. 1, it can be provided that the fan 9 is the suction type, and is located outside the compartment 5, performing an extraction and non-thrusting function of the air flows F2.

According to some embodiments, the lamps 7 can be selected from different types of light sources which emit different inspection lights, and the choice between them is left to the person of skill in the art, according to the best use of the apparatus 1.

According to some embodiments, the lamps 7 comprise LED (Light Emitting Diode) devices, suitable to provide a light beam which has sufficient intensity to pass through the thickness of the sample 2 to be inspected, but without producing excessive heat, which could damage and deteriorate the sample 2.

According to some embodiments, the light used for the inspections can be the ultraviolet type.

According to other embodiments, the light used for the inspections can be the type known as Wood's light.

According to other embodiments, the light used for the inspections can be a blue light of another type.

The apparatus 1 comprises, mounted inside the chamber 14, two visual inspection devices 15a, 15b suitable to acquire an image of the sample 2 to be inspected.

Each visual inspection device 15a, 15b is connected to a display screen 16, typically positioned in an observation station for an operator, indicated schematically by the reference number 17.

The box-like frame 13 is configured to generate a dark chamber 14 for the visual inspection device 15 in order to prevent disturbances by external light sources.

According to some embodiments, the walls of the chamber 14 are black and opaque to absorb the light which goes beyond the plane 3 and the sample 2 and minimize possible reflections which could disturb the generation of the shaded areas on the surface of the product to be inspected.

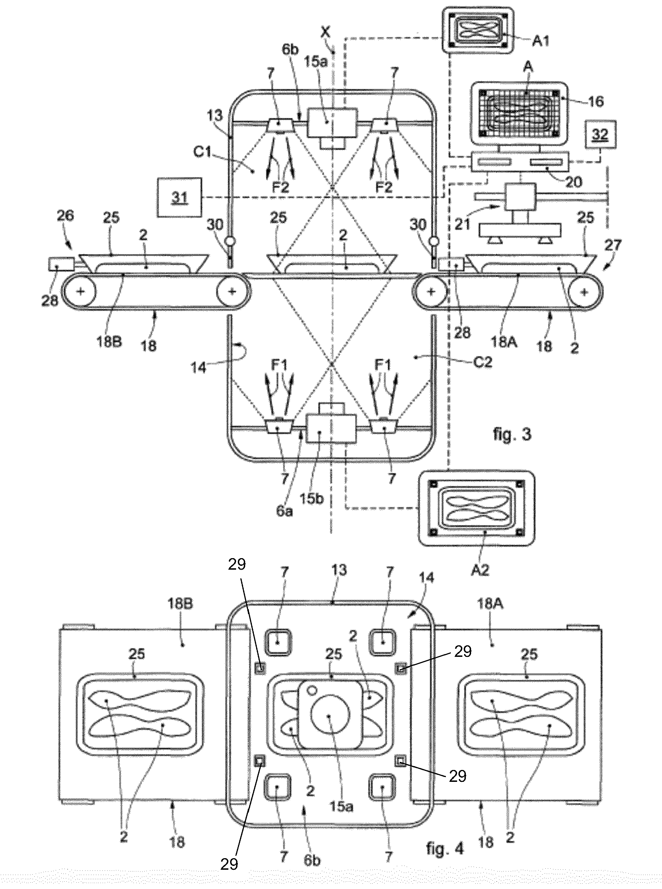

According to some embodiments, for example described with reference to fig. 3, the support plane 3 cooperates with the box-like frame 13 to define an upper dark chamber C1 and a lower dark chamber C2.

According to these embodiments, it can be provided that the box-like frame 13 has sizes correlated to that of the plane 3, and is possibly provided with doors 30 which can be opened and closed to allow the passage of the packages 25 inside and outside the chamber 14. In this way, the optimal light conditions for the acquisition of the images are guaranteed, eliminating possible external interferences or reflections.

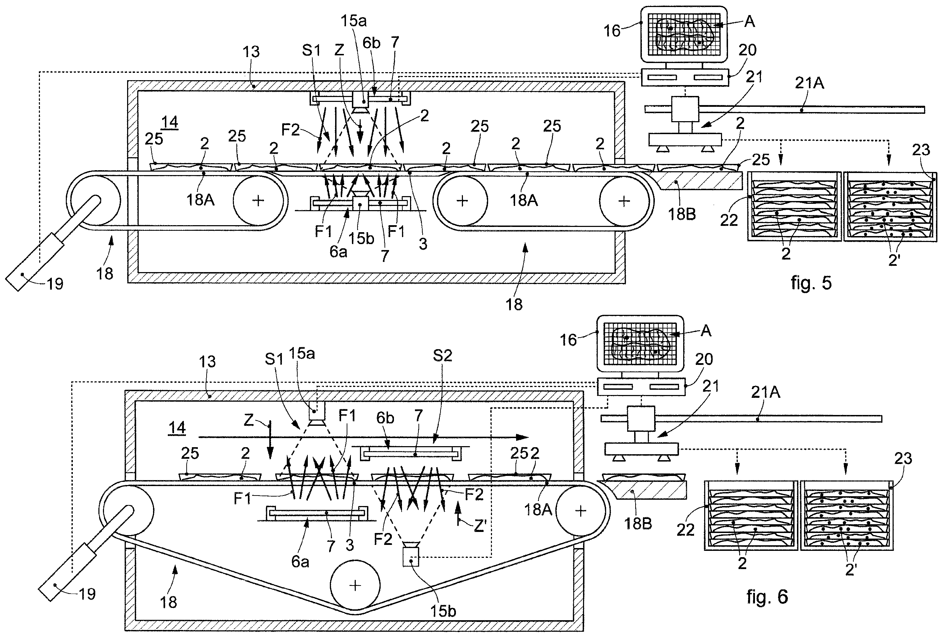

According to possible variant embodiments, for example described with reference to figs. 5 and 6, the box-like frame 13 has sizes larger than the plane 3.

According to some embodiments, the visual inspection device 15a, 15b can comprise an image acquisition device, for example a camera, or a video camera, suitable to acquire an image A1, A2 of a sample 2.

Each visual acquisition device 15a, 15b is disposed on the opposite side of the support plane 3 with respect to a corresponding light source 6a, 6b, so as to cooperate with it and acquire the backlit image A of the sample 2.

The apparatus 1 therefore comprises at least two inspection stations S1, S2, each consisting of a light source 6a, 6b and a corresponding visual acquisition device 15a, 15b.

According to some embodiments, for example described with reference to figs. 1, 3-5, it can be provided that the inspection stations S1, S2 are located in a same position in a direction of feed D of the samples 2, inverted one with respect to the other.

For example, it can be provided that the visual inspection devices 15a, 15b are positioned opposite each other and aligned along the same central axis X passing through substantially the center of the plane 3.

According to other embodiments, the light sources 6a, 6b each comprise two or more LED lighting devices 7, for example four as shown in fig. 4, disposed around the central axis X and in opposite pairs.

The visual inspection device 15, if required, can have a lens equipped with a filter which reproduces a Cartesian grid to divide the samples 2 into visual inspection zones.

The apparatus 1 comprises a processing unit, for example a computer 20, a processor, or other processing device, configured to analyze and process the images A1, A2 acquired by each visual inspection device 15a, 15b.

According to some embodiments, the computer 20 can receive the image A1, A2 acquired and divide it into a plurality of cells, for example ordered according to a matrix of rows and columns, and analyze each cell to identify the presence of possible parasites.

According to some embodiments, it can be provided that the computer 20 can identify the presence of possible parasites by analyzing the color of the cells in the image A1, A2 to identify possible zones or anomalies of different color in the sample 2. For example, there may be a zone in the image with a lighter, or darker, color than the surrounding median color, which could be caused by the presence of a parasite, a bone, or other foreign body.

The computer 20 is configured to selectively activate and deactivate the light sources 6a, 6b and the visual inspection devices 15a, 15b, respectively cooperating with it to obtain the images of the samples 2. In particular, the computer 20 can activate the light source 6a and the visual inspection device 15a of the first station S1 to obtain a first image and subsequently deactivate them and activate the light source 6b and the visual inspection device 15b of the second station S2 to obtain the second image.

According to other embodiments, the computer 20 can also be configured to process the images A1, A2 acquired by the individual visual inspection devices 15a, 15b on a given sample 2 and generate a single image A by overlapping them, and make it visible on the screen 16.

According to the invention, see fig. 4, the plane 3 comprises opaque reference elements 29, that is, such as to intercept at least part of the light beam. In this way, they can be photographed by the visual inspection devices 15a, 15b together with the samples 2 and can be used by the computer 20 to generate the overlapped image A. The reference elements 29 can be, for example, a symbol with a defined shape, like a circle, a cross, a triangle, a square, or other.

With reference to fig. 3, another embodiment of the apparatus 1 is shown, specifically disposed for the continuous inspection of the samples 2.

In this case too, for the sake of clarity, the elements common to the previously described versions of the apparatus 1 are indicated with the same numerical references.

The apparatus 1 comprises feed means 26 configured to feed the samples 2 to the chamber 14 and removal means 27 configured to remove the viewed samples 2 from the chamber 14.

According to some embodiments, the feed means 26 can comprise a conveyor belt 18 suitable to continuously feed the samples 2 on the transparent plane 3 in order to be inspected.

According to possible solutions, the conveyor belt 18 can feed the packages, or the trays 25, aligned one in succession to the other toward the transparent plane 3 so that each tray 25 thrusts the one immediately preceding it.

According to some embodiments, the removal means 27 can also comprise a conveyor belt 18.

According to variant embodiments, for example visible in figs. 3-4, two conveyor belts 18 can be provided, located respectively one upstream and the other downstream of the transparent plane 3, respectively to feed the trays 25 with the samples 2 to be inspected, and recover them after inspection.

According to some embodiments, each conveyor belt 18 is driven by a motor-gearmotor assembly 19 which is connected to the computer 20, to which both the visual inspection device 15 and the display screen 16 are also connected, so that it is possible to regulate the speed of feed of the conveyor belt or belts 18 as a function of the nature of the samples 2 and the inspection capacity of the operators.

According to some embodiments, a gripping device 21 can also be connected to the computer 20 which, according to the result of the inspections displayed on the display screen 16, removes the samples 2 from a plane 18B located at the exit front of the conveyor 18 and, moving on a guide 21A, deposits them in containers 22 and 23, dividing them between samples 2 free of parasites and samples 2' infested by parasites.

According to possible variant embodiments described with reference to fig. 1, the feed means 26 and/or the removal means 27 can comprise piston devices 28, articulated arms, or similar devices, configured respectively to remove a tray 25 containing the samples 2 to be inspected and position it on the plane 3, or to remove it from the latter.

According to other embodiments, the apparatus 1 can comprise reading devices 31 suitable to read a code provided on the packages, and to communicate it to the processing unit 20.

The codes can be, for example, barcodes, QR codes, or infrared codes, containing information on the food product of the sample 2, the packaging or expiry date, or other.

According to some embodiments, the apparatus 1 comprises a memory unit 32 connected to the processing unit 20 and configured to memorize the codes read by the reading devices 31 and the images acquired by the visual inspection devices 15a, 15b.

With reference to fig. 6, another possible embodiment of the apparatus 1 is shown, in which the transparent plane 3 is defined by a conveyor belt 18 made of a highly transparent flexible material, on whose upper active branch 18A a succession of samples 2 to be inspected rests.

As can be seen, a lamp 7 is located under the active branch 18A, and irradiates both the latter and also the samples 2 from the bottom upward, making them inspectable through transparency, and another lamp 7 is located offset with respect to the first lamp with respect to a direction of feed D, above the active branch 18A and irradiating from the top downward.

According to some embodiments, which can be combined with the previous ones, it can be provided that the apparatus 1 comprises the two inspection stations S1, S2 located in series one with the other.

In the example shown, it can be provided that the samples 2 are translated from the first inspection station S1 defined by a lamp 7 and a display unit 15a, to the second inspection station S2 defined by the other lamp 7 and by the other display unit 15b, directly by means of the same active branch 18A of the conveyor belt 18.

Also according to the embodiment given by way of example described with reference to fig. 6, the motor-gearmotor assembly 19, the visual inspection devices 15a and 15b, the display screen 16 and the gripping device 21 are operatively connected to the computer 20 to regulate the speed of feed of the conveyor 18 according to the capacity of inspection of the operators.

The functioning of the invention can intuitively be understood from the above description.

In general, it should be underlined that according to the method for the inspection of food products according to the invention and which is implemented with all the versions of the apparatus 1 described above, the observation of fish samples 2, but also of other foods, occurs through transparency, that is, observing the samples 2 in at least two directions of observation Z, Z' from the opposite side thereof with respect to the one which is irradiated by the inspection light sources 6a, 6b which pass through the transparent support plane 3.

According to the method and the apparatus 1 for the inspection of food products, the samples 2 are observed during the inspection in two opposite directions, precisely the direction Z and an opposite direction Z'.

This allows to prevent refraction phenomena that could hide the presence of parasites in the samples 2 inspected.

According to this embodiment, it can be provided to acquire an image of the samples 2 with two inspection devices 15a, 15b disposed in opposite directions with respect to the support plane 3, and to process the two images acquired, possibly overlapping one above the other, by means of the computer 20.

The acquisition of the images provides to acquire, together with the image of the sample 2, also an image of opaque reference elements 29 provided on the plane 3.

The images can be processed to generate an overlapping image of the first and second images according to the reference elements 29 acquired.

According to some embodiments, the method provides to reverse one of either said first image or said second image and to enlarge or reduce one or the other until the corresponding reference elements 29 are made to match.

According to some embodiments, the presence of the cooling fan 9 prevents the intensities of the light emitted by the inspection lamps 7 from altering the raw state of the samples 2 during the inspections, for example by triggering an initial cooking.

The presence of visual inspection devices 15a and 15b allows to observe in detail and also at a distance the samples 2 during the inspection to which they are subjected.

The management program that is loaded on the computer 20 can, if required, also take detailed photographs of parts of the samples 2 and produce paper printouts to also make comparisons in this way.

According to other embodiments, the method provides to read a code provided on the packages 25 and containing information relating to the sample 2 inside them and, at the end of processing, it provides to combine the images acquired with the code and memorize them in a memory unit 32.

According to other embodiments, a database of images of food products in which a parasite or a foreign body is present can be provided in the memory unit 32, and the processing unit 20 can be configured to generate a standard image of a parasite, or of a foreign body on the basis of the data memorized.

According to these solutions, a software program can be provided in the processing unit 20 which is suitable to compare the standard image with the images acquired A1, A2, A, and possibly highlight the suspicious areas and make them visible to the operator on the screen 16, in order to facilitate their inspection.

In practice it has been found that the invention achieves the intended purposes.

In practical implementation, it is possible to use any materials whatsoever, having any shape or size, according to requirements, without departing from the field of protection of the present invention.

It is clear that modifications and/or additions of parts may be made to the method and apparatus 1 as described heretofore, without departing from the field and scope of the present invention.

It is also clear that, although the present invention has been described with reference to some specific examples, a person of skill in the art shall certainly be able to achieve many other equivalent forms of method and apparatus 1 for the inspection of fish products, having the characteristics as set forth in the claims and hence all coming within the field of protection defined thereby.

## Claims

1. Apparatus for the inspection of food product samples (2), in particular packaged raw fish products (25), comprising:
- a box-like frame (13) which delimits an inspection chamber (14);
- feed means (26) configured to feed the samples (2) to be inspected into said chamber (14);
- removal means configured to remove the inspected samples (2) from said chamber (14);
- a transparent support plane (3), disposed in said inspection chamber (14) and aligned with said feed and removal means and configured to support at least one sample (2);
- at least two light sources (6a, 6b) disposed on opposite sides of the plane (3) and each configured to emit a beam of light toward said at least one sample (2) supported on said plane (3);
- at least two visual inspection devices (15a, 15b) disposed on opposite sides of said plane (3), each configured to cooperate with one of said light sources (6a, 6b) in an opposite position to acquire a backlit image of the sample (2);
- a processing unit (20) connected to said visual inspection devices (15a, 15b) and configured to alternatively activate or deactivate said visual inspection devices (15a, 15b) and said light source (6a, 6b) cooperating reciprocally, and process the images acquired;
- a display device (16) configured to display said images acquired,
**characterized in that** it comprises:
- opaque reference elements (29) provided on said plane (3), able to be acquired by said visual inspection devices (15a, 15b) together with said backlit images of said food product (2), wherein said processing unit (20) is configured to generate an overlapping image of said first image and said second image according to said acquired reference elements.

2. Inspection apparatus as in claim 1, **characterized in that** said visual inspection devices (15a, 15b) are positioned opposite one another and aligned along a same central axis (X) passing through substantially the center of said plane (3).

3. Inspection apparatus as in claim 1 or 2, **characterized in that** said light sources (6a, 6b) each comprise two or more led illumination devices (7) opposite in pairs and disposed around said central axis (X).

4. Inspection apparatus as in claim 1, **characterized in that** said visual inspection devices (15a, 15b) are positioned offset one with respect to the other in a direction of feed (D) of said samples (2), and said light sources (6a, 6b) are each disposed in a coordinated position opposite to one of said visual inspection devices (15a, 15b) with respect to said plane (3).

5. Inspection apparatus as in any claim hereinbefore, **characterized in that** the internal walls of said box-like frame (13) are black and opaque, suitable to absorb the light that passes through said plane (3) and said sample (2), and to minimize possible reflections.

6. Inspection apparatus as in any claim hereinbefore, **characterized in that** upstream of said chamber (14) it comprises reading devices (31) suitable to read a code provided on said packages (25), carrying information relating to the samples (2).

7. Inspection apparatus as in any claim hereinbefore, **characterized in that** it comprises a memory unit (32) and said processing unit (20) is configured to receive the data read by the reading devices (31), combine them with said images acquired and memorize them in said memory unit (32).

8. Method for the inspection of food product samples (2), in particular raw fish products in packages (25), comprising:
- feeding a plurality of samples (2) to be inspected by feed means (26) into an inspection chamber (14) delimited by a box-like frame (13);
- inserting at least one sample (2) in said inspection chamber (14) resting on a transparent plane (3);
- illuminating said sample (2) with a first light source (6a) located underneath said plane (3) and acquiring a first backlit image of said sample (2) with a first visual inspection device (15a) disposed above said plane (3);
- illuminating said sample (2) with a second light source (6b) located above said plane (3) and acquiring a second backlit image of said sample (2) with a second visual inspection device (15b) disposed below said plane (3);
- processing said images acquired in order to recognize a parasite or foreign body in said sample (2) and making them visible to an operator on a display device (16);
**characterized in that** the acquisition of said images provides to also acquire, together with the backlit image of said sample (2), an image of opaque reference elements (29) provided on said plane (3) and said processing provides to generate an overlapping image of said first image and said second image according to said reference elements acquired.

9. Method as in claim 8, **characterized in that** it provides to reverse one of either said first image or second image and enlarge or reduce one or the other of said images until the respective reference elements are made to match, in order to generate an overlapping image and facilitate the identification of possible parasites or foreign bodies.

10. Method as in claim from 8 or 9, **characterized in that** it provides to read a code provided on said packages (25) and carrying information relating to the sample (2) to be inspected contained therein and, at the end of the processing it provides to combine said images acquired with said code and memorize them in a memory unit (32).

11. Method as in any claim from 8 to 10, **characterized in that** it provides to generate a standard image of a parasite, or a foreign body, on the basis of a plurality of images of food products in which a parasite or a foreign body is present, to compare the standard image with the images acquired, and to highlight the suspect areas making them visible to an operator on the screen (16), so as to facilitate the inspection thereof.

## Patentansprüche

1. Vorrichtung für die Untersuchung von Lebensmittelproduktproben (2), insbesondere von verpackten rohen Fischprodukten (25), aufweisend:
- einen kastenartigen Rahmen (13), der eine Untersuchungskammer (14) begrenzt,
- Zuführmittel (26), die konfiguriert sind, um die zu untersuchenden Proben (2) in die Kammer (14) zuzuführen,
- Entnahmemittel, die konfiguriert sind, um die untersuchten Proben (2) aus der Kammer (14) zu entnehmen,
- eine transparente Stützebene (3), die in der Untersuchungskammer (14) angeordnet und mit den Zuführ- und Entnahmemitteln ausgerichtet ist und konfiguriert ist, um mindestens eine Probe (2) abzustützen,
- mindestens zwei Lichtquellen (6a, 6b), die auf gegenüberliegenden Seiten der Ebene (3) angeordnet und jeweils konfiguriert sind, um einen Lichtstrahl in Richtung zu der mindestens einen auf der Ebene (3) abgestützten Probe (2) zu emittieren,
- mindestens zwei visuelle Untersuchungsvorrichtungen (15a, 15b), die auf gegenüberliegenden Seiten der Ebene (3) angeordnet sind, die jeweils konfiguriert sind, um mit einer der Lichtquellen (6a, 6b) in einer gegenüberliegenden Position zusammenzuwirken, um ein hinterleuchtetes Bild der Probe (2) zu erfassen,
- eine Verarbeitungseinheit (20), die mit den visuellen Untersuchungsvorrichtungen (15a, 15b) verbunden ist und konfiguriert ist, um die visuellen Untersuchungsvorrichtungen (15a, 15b) und die Lichtquelle (6a, 6b), die wechselseitig zusammenwirken, alternativ zu aktivieren oder zu deaktivieren, und die erfassten Bilder zu verarbeiten,
- eine Anzeigevorrichtung (16), die konfiguriert ist, um die erfassten Bilder anzuzeigen, **dadurch gekennzeichnet, dass** sie aufweist:
- undurchsichtige Referenzelemente (29), die auf der Ebene (3) vorgesehen sind, die geeignet sind, um von den visuellen Untersuchungsvorrichtungen (15a, 15b) zusammen mit den hinterleuchteten Bildern des Lebensmittelprodukts (2) erfasst zu werden, wobei die Verarbeitungseinheit (20) konfiguriert ist, um ein überlappendes Bild des ersten Bildes und des zweiten Bildes gemäß den erfassten Referenzelementen zu erzeugen.

2. Untersuchungsvorrichtung gemäß Anspruch 1, **dadurch gekennzeichnet, dass** die visuellen Untersuchungsvorrichtungen (15a, 15b) einander gegenüber positioniert und entlang einer gleichen zentralen Achse (X) ausgerichtet sind, die im Wesentlichen durch die Mitte der Ebene (3) verläuft.

3. Untersuchungsvorrichtung gemäß Anspruch 1 oder 2, **dadurch gekennzeichnet, dass** die Lichtquellen (6a, 6b) jeweils zwei oder mehr paarweise gegenüberliegende und um die zentrale Achse (X) herum angeordnete LED-Beleuchtungsvorrichtungen (7) aufweisen.

4. Untersuchungsvorrichtung gemäß Anspruch 1, **dadurch gekennzeichnet, dass** die visuellen Untersuchungsvorrichtungen (15a, 15b) in einer Zuführrichtung (D) der Proben (2) zueinander versetzt positioniert sind, und die Lichtquellen (6a, 6b) in Bezug auf die Ebene (3) jeweils in einer koordinierten Position gegenüber einer der visuellen Untersuchungsvorrichtungen (15a, 15b) angeordnet sind.

5. Untersuchungsvorrichtung gemäß irgendeinem vorhergehenden Anspruch, **dadurch gekennzeichnet, dass** die Innenwände des kastenartigen Rahmens (13) schwarz und undurchsichtig sind, geeignet sind, um das Licht zu absorbieren, das durch die Ebene (3) und die Probe (2) hindurchtritt, und mögliche Reflexionen zu minimieren.

6. Untersuchungsvorrichtung gemäß irgendeinem vorhergehenden Anspruch, **dadurch gekennzeichnet, dass** sie stromaufwärts der Kammer (14) Lesevorrichtungen (31) aufweist, die geeignet sind, einen auf den Verpackungen (25) bereitgestellten Code zu lesen, der Informationen bezüglich der Proben (2) trägt.

7. Untersuchungsvorrichtung gemäß irgendeinem vorhergehenden Anspruch, **dadurch gekennzeichnet, dass** sie eine Speichereinheit (32) aufweist und die Verarbeitungseinheit (20) konfiguriert ist, um die von den Lesevorrichtungen (31) gelesenen Daten zu empfangen, sie mit den erfassten Bildern zu kombinieren und sie in der Speichereinheit (32) zu speichern.

8. Verfahren zur Untersuchung von Lebensmittelproduktproben (2), insbesondere von rohen Fischprodukten in Verpackungen (25), aufweisend:
- Zuführen einer Mehrzahl von zu untersuchenden Proben (2) durch Zuführmittel (26) in eine Untersuchungskammer (14), die durch einen kastenartigen Rahmen (13) begrenzt ist,
- Einsetzen von mindestens einer Probe (2) in die Untersuchungskammer (14), die auf einer transparenten Ebene (3) aufliegt,
- Beleuchten der Probe (2) mit einer ersten Lichtquelle (6a), die sich unterhalb der Ebene (3) befindet, und Erfassen eines ersten hinterleuchteten Bildes der Probe (2) mit einer ersten visuellen Untersuchungsvorrichtung (15a), die über der Ebene (3) angeordnet ist,
- Beleuchten der Probe (2) mit einer zweiten Lichtquelle (6b), die sich über der Ebene (3) befindet, und Erfassen eines zweiten hinterleuchteten Bildes der Probe (2) mit einer zweiten visuellen Untersuchungsvorrichtung (15b), die unter der Ebene (3) angeordnet ist,
- Verarbeiten der erfassten Bilder, um einen Parasiten oder Fremdkörper in der Probe (2) zu erkennen, und Sichtbarmachen derselben für einen Anwender auf einer Anzeigevorrichtung (16),
**dadurch gekennzeichnet, dass** das Erfassen der Bilder vorsieht, zusammen mit dem hinterleuchteten Bild der Probe (2) auch ein Bild von undurchsichtigen Referenzelementen (29) zu erfassen, die auf der Ebene (3) vorgesehen sind, und das Verarbeiten vorsieht, ein überlappendes Bild des ersten Bildes und des zweiten Bildes gemäß den erfassten Referenzelementen zu erzeugen.

9. Verfahren gemäß Anspruch 8, **dadurch gekennzeichnet, dass** es vorsieht, eines von entweder dem ersten Bild oder dem zweiten Bild umzukehren und eines oder das andere der Bilder zu vergrößern oder zu verkleinern, bis die jeweiligen Referenzelemente in Übereinstimmung gebracht sind, um ein überlappendes Bild zu erzeugen und die Identifizierung möglicher Parasiten oder Fremdkörper zu erleichtern.

10. Verfahren gemäß Anspruch 8 oder 9, **dadurch gekennzeichnet, dass** es vorsieht, einen Code zu lesen, der auf den Verpackungen (25) bereitgestellt ist und Informationen bezüglich der darin enthaltenen zu untersuchenden Probe (2) trägt, und am Ende des Verarbeitens vorsieht, die erfassten Bilder mit dem Code zu kombinieren und sie in einer Speichereinheit (32) zu speichern.

11. Verfahren gemäß irgendeinem der Ansprüche 8 bis 10, **dadurch gekennzeichnet, dass** es vorsieht, ein Standardbild eines Parasiten oder eines Fremdkörpers auf der Basis einer Mehrzahl von Bildern von Lebensmittelprodukten zu erzeugen, in denen ein Parasit oder ein Fremdkörper vorhanden ist, das Standardbild mit den erfassten Bildern zu vergleichen und die verdächtigen Bereiche hervorzuheben, wobei sie für einen Anwender auf dem Bildschirm (16) sichtbar gemacht werden, um so die Untersuchung davon zu erleichtern.

## Revendications

1. Appareil pour l'inspection d'échantillons de produits alimentaires (2), en particulier de produits de poisson crus emballés (25), comprenant :
- un cadre en forme de boîte (13) qui délimite une chambre d'inspection (14) ;
- des moyens d'alimentation (26) configurés pour alimenter les échantillons (2) à inspecter dans ladite chambre (14) ;
- des moyens d'alimentation (26) configurés pour alimenter les échantillons (2) à inspecter dans ladite chambre (14) ;
- un plan de support transparent (3), disposé dans ladite chambre d'inspection (14) et aligné avec lesdits moyens d'alimentation et de retrait et configuré pour supporter au moins un échantillon (2) ;
- au moins deux sources lumineuses (6a, 6b) disposées sur des côtés opposés du plan (3) et chacune configurée pour émettre un faisceau de lumière vers ledit au moins un échantillon (2) supporté sur ledit plan (3) ;
- au moins deux dispositifs d'inspection visuelle (15a, 15b) disposés sur des côtés opposés dudit plan (3), chacun étant configuré pour coopérer avec l'une desdites sources lumineuses (6a, 6b) dans une position opposée pour acquérir une image rétroéclairée de l'échantillon (2) ;
- une unité de traitement (20) connectée auxdits dispositifs d'inspection visuelle (15a, 15b) et configurée pour activer ou désactiver alternativement lesdits dispositifs d'inspection visuelle (15a, 15b) et ladite source lumineuse (6a, 6b) coopérant réciproquement, et traiter les images acquises ;
- un dispositif d'affichage (16) configuré pour afficher lesdites images acquises,
**caractérisé en ce qu'il comprend :**
- des éléments de référence opaques (29) prévus sur ledit plan (3), pouvant être acquis par lesdits dispositifs d'inspection visuelle (15a, 15b) conjointement avec lesdites images rétroéclairées dudit produit alimentaire (2), où ladite unité de traitement (20) est configurée pour générer une image chevauchante de ladite première image et de ladite seconde image conformément auxdits éléments de référence acquis.

2. Appareil d'inspection selon la revendication 1, **caractérisé en ce que** lesdits dispositifs d'inspection visuelle (15a, 15b) sont positionnés opposés entre eux et alignés le long d'un même axe central (X) passant sensiblement par le centre dudit plan (3).

3. Appareil d'inspection selon la revendication 1 ou 2, **caractérisé en ce que** lesdites sources lumineuses (6a, 6b) comprennent chacune deux ou plusieurs dispositifs d'éclairage LED (7) opposés par paires et disposés autour dudit axe central (X).

4. Appareil d'inspection selon la revendication 1, **caractérisé en ce que** lesdits dispositifs d'inspection visuelle (15a, 15b) sont positionnés de manière décalée l'un par rapport à l'autre dans une direction d'alimentation (D) desdits échantillons (2), et lesdites sources lumineuses (6a, 6b) sont chacune disposées dans une position coordonnée opposée à l'un desdits dispositifs d'inspection visuelle (15a, 15b) par rapport audit plan (3).

5. Appareil d'inspection selon l'une quelconque des revendications précédentes, **caractérisé en ce que** les parois internes dudit cadre en forme de boîte (13) sont noires et opaques, appropriées pour absorber la lumière qui passe à travers ledit plan (3) et ledit échantillon (2), et pour minimiser les réflexions possibles.

6. Appareil d'inspection selon l'une quelconque des revendications précédentes, **caractérisé en ce que**, en amont de ladite chambre (14), il comprend des dispositifs de lecture (31) adaptés pour lire un code prévu sur lesdits emballages (25), portant des informations relatives aux échantillons (2).

7. Appareil d'inspection selon l'une quelconque des revendications précédentes, **caractérisé en ce qu'**il comprend une unité de mémoire (32) et ladite unité de traitement (20) est configurée pour recevoir les données lues par les dispositifs de lecture (31), les combiner avec lesdites images acquises et les mémoriser dans ladite unité de mémoire (32).

8. Procédé d'inspection d'échantillons de produits alimentaires (2), en particulier de produits de poisson crus dans des emballages (25), comprenant :
- l'alimentation d'une pluralité d'échantillons (2) à inspecter par des moyens d'alimentation (26) dans une chambre d'inspection (14) délimitée par un cadre en forme de boîte (13) ;
- l'introduction d'au moins un échantillon (2) dans ladite chambre d'inspection (14) reposant sur un plan transparent (3) ;
- l'éclairage dudit échantillon (2) avec une première source lumineuse (6a) située sous ledit plan (3) et l'acquisition d'une première image rétroéclairée dudit échantillon (2) avec un premier dispositif d'inspection visuelle (15a) disposé au-dessus dudit plan (3) ;
- l'éclairage dudit échantillon (2) avec une seconde source lumineuse (6b) située au-dessus dudit plan (3) et l'acquisition d'une seconde image rétroéclairée dudit échantillon (2) avec un second dispositif d'inspection visuelle (15b) disposé au-dessous dudit plan (3) ;
- le traitement desdites images acquises afin de reconnaître un parasite ou un corps étranger dans ledit échantillon (2) et les rendre visibles par un opérateur sur un dispositif d'affichage (16) ;
**caractérisé en ce que** l'acquisition desdites images permet également d'acquérir, conjointement avec l'image rétroéclairée dudit échantillon (2), une image d'éléments de référence opaques (29) prévus sur ledit plan (3) et ledit traitement permet de générer une image chevauchante de ladite première image et de ladite seconde image selon lesdits éléments de référence acquis.

9. Procédé selon la revendication 8, **caractérisé en ce qu'**il prévoit d'inverser l'une de ladite première image ou de ladite seconde image et d'agrandir ou de réduire l'une ou l'autre desdites images jusqu'à ce que les éléments de référence respectifs soient amenés à correspondre, afin de générer une image chevauchante et de faciliter l'identification de parasites éventuels ou de corps étrangers.

10. Procédé selon la revendication 8 ou 9, **caractérisé en ce qu'**il prévoit de lire un code prévu sur lesdits emballages (25) et transportant des informations relatives à l'échantillon (2) à inspecter qu'il contient et, à la fin du traitement, il prévoit de combiner lesdites images acquises avec ledit code et de les mémoriser dans une unité de mémoire (32).

11. Procédé selon l'une quelconque des revendications 8 à 10, **caractérisé en ce qu'**il prévoit de générer une image standard d'un parasite, ou d'un corps étranger, sur la base d'une pluralité d'images de produits alimentaires dans lesquels un parasite ou un corps étranger est présent, de comparer l'image standard avec les images acquises, et de mettre en évidence les zones suspectes les rendant visibles pour un opérateur sur l'écran (16), de manière à faciliter l'inspection de ceux-ci.
